# EUROPEAN PATENT APPLICATION

(11) **EP 4 342 474 A1**
(43) Date of publication of application: **27.03.2024**
(21) Application number: 21940940.6
(22) Date of filing: 25.10.2021
(51) Int. Cl.: A61K 31/575, A61P 21/00, A23L 33/10

(54) **COMPOSITION COMPRISING INOTODIOL FOR PREVENTION OR TREATMENT OF MUSCULAR DISEASE**

(30) Priority: 21.05.2021 KR 20210065626
(71) Applicant: Animuscure Inc., Suwon-si, Gyeonggi-do 16419 (KR)
(72) Inventor: LEE, Sang-Jin, Seoul 04747 (KR); YOU, Chang-Lim, Anyang-si Gyeonggi-do 16362 (KR); BAE, Ju-Hyeon, Suwon-si, Gyeonggi-do 164221 (KR)
(74) Representative: Dehns
(86) International application number: PCT/KR2021/015026
(87) International publication number: WO 2022/244929

(57) **Abstract**

The present invention relates to a composition comprising an inotodiol compound for prevention, alleviation, or treatment of muscular diseases. The composition increases skeletal muscle mass and promotes the regeneration of muscle fibers through self-renewal acceleration of muscle stem cells and differentiation promotion of myoblasts, and reinforces muscular strength by inducing the activation of mitochondrial functions, whereby the composition has prophylactic and therapeutic effects on various muscular diseases and an effect of increasing motor ability by reinforcing muscular strength.

## Description

### [Technical Field]

The present invention relates to a composition for preventing, alleviating or treating a muscular disease comprising inotodiol.

### [Background Art]

Muscles are an important part of body functions such as energy metabolism, exercise capacity, and the like, and may be damaged or weakened by various factors such as sarcopenia caused due to aging, muscular atrophy caused due to a nutritional imbalance or lack of exercise, other diseases such as cancer, aging, and the like.

Sarcopenia, which is a major disease that damages muscles, is a disease in which muscle strength decreases as muscle mass (i.e., skeletal muscle mass) decreases with aging. The most significant feature of sarcopenia is a decrease in muscle mass, and the type of muscle fiber may change. While type I and type II muscle fibers decrease at a similar rate with aging, the thickness of the type I muscle fibers decreases more significantly in patients with sarcopenia. It has been reported that such sarcopenia causes muscle weakness and functional impairments that occur among the elderly (Roubenoff R., Can. J. Appl. Physiol. 26, 78-89, 2001).

Also, muscle atrophy is caused by a nutritional deficiency or long-term muscle disuse. In this case, muscle atrophy develops when the balance between normal protein synthesis and degradation is disrupted so that proteins are degraded in muscle.

Various therapeutic methods for fundamentally treating these muscular diseases are being developed. In particular, a therapeutic method using a mechanism that strengthens muscles by promoting the differentiation of muscle cells from stem cells or promotes the regeneration of muscles has been proposed. Because this method may be fundamentally used to treat muscular diseases, there is a need for research on various therapeutic substances that enable this treatment.

The present inventors have conducted research on compounds having an effect of alleviating muscular diseases through the above-described effects from various compounds. Therefore, the present invention has been completed based on these results.

### [Disclosure]

### [Technical Problem]

The present inventors have found that inotodiol promotes the differentiation of myoblasts to increase muscle regeneration and muscle mass, and enhances energy metabolism in muscle to enhance muscle strength, thereby having a therapeutic effect on muscular diseases such as muscular dystrophy. Therefore, the present invention has been completed based on these facts.

Therefore, it is an object of the present invention to provide a pharmaceutical composition for preventing or treating a muscular disease, comprising inotodiol or a pharmaceutically acceptable salt thereof.

It is another object of the present invention to provide a health functional food composition for preventing or alleviating a muscular disease, comprising inotodiol or a sitologically acceptable salt thereof.

It is still another object of the present invention to provide an animal feed composition for preventing or alleviating a muscular disease, comprising inotodiol or a salt thereof.

It is yet another object of the present invention to provide a composition for promoting muscle regeneration capacity or increasing muscle mass by enhancing the self-renewal capacity of muscle stem cells and the differentiation capacity of myoblasts, which comprises an inotodiol compound.

It is yet another object of the present invention to provide a composition for improving muscle function, comprising an inotodiol compound.

It is yet another object of the present invention to provide a composition for enhancing muscle strength, comprising inotodiol.

It is yet another object of the present invention to provide a composition for alleviating muscle fibrosis, comprising inotodiol.

It is yet another object of the present invention to provide a composition for improving exercise performance ability, comprising inotodiol.

It is yet another object of the present invention to provide a method of preventing, alleviating, or treating a muscular disease, which copmrise: administering inotodiol or a pharmaceutically acceptable salt thereof to a subject.

It is yet another object of the present invention to provide a use of the inotodiol or a pharmaceutically acceptable salt thereof for the prevention, alleviation, or treatment of a muscular disease.

### [Technical Solution]

To achieve the above objects, according to an aspect of the present invention, there is provided a pharmaceutical composition for preventing or treating a muscular disease, comprising inotodiol or a pharmaceutically acceptable salt thereof.

According to another aspect of the present invention, there is provided a health functional food composition for preventing or alleviating a muscular disease, comprising inotodiol or a sitologically acceptable salt thereof.

According to still another aspect of the present invention, there is provided an animal feed composition for preventing or alleviating a muscular disease, comprising an inotodiol compound or a salt thereof.

To achieve other objects, according to an aspect of the present invention, there is provided a composition for promoting muscle regeneration capacity or increasing muscle mass by enhancing the self-renewal capacity of muscle stem cells and the muscle differentiation capacity of myoblasts, which ccomprises an inotodiol compound.

According to another aspect of the present invention, there is provided a composition for improving muscle function, comprising an inotodiol compound.

According to still another aspect of the present invention, there is provided a composition for enhancing muscle strength, comprising inotodiol.

According to yet another aspect of the present invention, there is provided a composition for alleviating muscle fibrosis, comprising inotodiol.

According to yet another aspect of the present invention, there is provided a composition for improving exercise performance ability, comprising inotodiol.

According to yet another aspect of the present invention, there is provided a method of preventing, alleviating, or treating a muscular disease, which comprise: administering inotodiol or a pharmaceutically acceptable salt thereof to a subject.

To achieve other objects, according to an aspect of the present invention, there is provided a use of inotodiol or a pharmaceutically acceptable salt thereof for the prevention, alleviation, or treatment of a muscular disease.

### [Advantageous Effects]

The present invention relates to a composition for preventing, alleviating, or treating a muscular disease, which comprises an inotodiol compound. The composition increases muscle mass and muscle fiber regeneration by promoting the differentiation of myoblasts, and enhances muscle strength by inducing the activation of mitochondrial functions, and thus has a therapeutic effect on various muscular diseases. Accordingly, the composition can be used as a pharmaceutical or health functional food.

### [Description of Drawings]

FIGS. 1A and 1B show the results showing the degrees of differentiation of mouse myoblasts (C2C12) by the administration of inotodiol at different concentrations. Statistics are expressed as *p < 0.05, **p < 0.01, and ***p < 0.001 through a one-way ANOVA test.
FIG. 2 shows the results of confirming that the luciferase activity for PGC-1α in inotodiol-administered myoblasts (C2C12) increases in a concentration-dependent manner. Statistics are expressed as *p < 0.05, **p < 0.01, and ***p < 0.001 through a one-way ANOVA test.
FIG. 3 shows the results of confirming an mRNA expression level for PGC-1α in inotodiol-administered myoblasts (C2C12) through qRT-PCR. Statistics are expressed as *p < 0.05, **p < 0.01, and ***p < 0.001 via a Student t-test.
FIG. 4 shows the results of observing the expression of mitochondrial activity markers in muscle by treating myoblasts (C2C12) with inotodiol at each concentration.
FIG. 5 shows the results of confirming the effect of increasing the muscle mass (TA, EDL, SOL, GAS, respectively) of the mouse hind limbs by the administration of inotodiol in a mouse model in which muscle damage is induced by CTX. Here, FIG. 5A shows a change in body weight, and FIG. 5B shows changes in weight for a part of each hind limb muscle. Statistics are expressed as *p < 0.05, **p < 0.01, and ***p < 0.001 via a Student t-test.
FIGS. 6A to 6C shows the results of confirming the effect of regenerating muscle fibers and increasing a cross-sectional area (CSA) of the muscle fibers when inotodiol is administered to a mouse model in which muscle damage is induced by CTX. Statistics are expressed as *p < 0.05, **p < 0.01, and ***p < 0.001 via a Student t-test.
FIG. 7 shows the results of comparing the relative mRNA expression levels for each muscle fiber type when inotodiol is administered to mice in which muscle damage is induced by CTX. Statistics are expressed as *p < 0.05, **p < 0.01, and ***p < 0.001 via a Student t-test.
FIG. 8 shows the result of confirming the effect of increasing grip strength when inotodiol is administered to a mouse model in which muscle damage is induced. Statistics are expressed as *p < 0.05, **p < 0.01, and ***p < 0.001 via a Student t-test.
FIG. 9 shows the results of reducing blood glucose by the administration of inotodiol to a mouse model in which muscle damage is induced. Statistics are expressed as *p < 0.05, **p < 0.01, and ***p < 0.001 via a Student t-test.
FIGS. 10 to 12 show the process and results of BrdU analysis experiments to confirm the proliferation of muscle stem cells when inotodiol is administered after muscle damage is induced by CTX. Statistics are expressed as *p < 0.05, **p < 0.01, and ***p < 0.001 via a Student t-test.
FIG. 13 shows the results of comparing the relative mRNA expression levels of genes involved in the cell cycle when inotodiol is administered after muscle damage is induced by CTX. Statistics are expressed as *p < 0.05, **p < 0.01, and ***p < 0.001 via a Student t-test.
FIG. 14 shows the results of confirming the effect of enhancing the expression of genes involved in muscle growth when inotodiol is administered after muscle damage is induced by CTX. Statistics are expressed as *p < 0.05, **p < 0.01, and ***p < 0.001 via a Student t-test.
FIGS. 15A and 15B show the result of observing the effect of alleviating muscle fibrosis when inotodiol is administered after muscle damage is induced by CTX.
FIGS. 16 and 17 show the results of observing the changes in muscle strength and exercise performance ability when inotodiol was administered to a high-fat diet mouse model.
FIGS. 18 and 19 show the results of observing muscles, and the changes in size of muscle fibers and cross-sectional area of myotubes by the administration of inotodiol in a mouse model in which myoatrophy is induced by DEX. Statistics are expressed as *p < 0.05, **p < 0.01, and ***p < 0.001 through a one-way ANOVA test.
FIG. 20 shows the results of measuring changes in muscle mass and muscle strength after inotodiol is administered to normal mice for a long-term period of 2 months. Statistics are expressed as *p < 0.05, **p < 0.01 and, ***p < 0.001 via a Student t-test.
FIG. 21 shows the results of examining the expression levels of PGC1a and mitochondrial activity genes in normal mice to confirm mitochondrial activity in muscle. Statistics are expressed as *p < 0.05, **p < 0.01, and ***p < 0.001 via a Student t-test.
FIG. 22 and 23 show the results of observing changes in muscle and fat weights relative to a change in body weight in normal mice. Statistics are expressed as *p < 0.05, **p < 0.01, and ***p < 0.001 through a two-way ANOVA or Student t-test.

### [Best Mode]

The present invention relates to a pharmaceutical composition for preventing or treating a muscular disease, which comprises inotodiol or a pharmaceutically acceptable salt thereof.

The present invention relates to a health functional food composition for preventing or alleviating a muscular disease, which comprises inotodiol or a sitologically acceptable salt thereof.

The present invention relates to an animal feed composition for preventing or alleviating a muscular disease, which comprises an inotodiol compound or a salt thereof.

The present invention relates to a composition for promoting muscle regeneration capacity or increasing or muscle mass by enhancing the self-renewal capacity of muscle stem cells and the differentiation capacity of myoblasts, which comprises an inotodiol compound.

The present invention relates to a composition for improving muscle function, which comprises an inotodiol compound.

The present invention relates to a composition for enhancing muscle strength, which comprises inotodiol.

The present invention relates to a method of preventing, alleviating, or treating a muscular disease, which comprises: administering inotodiol or a pharmaceutically acceptable salt thereof to a subject.

The present invention relates to a use of inotodiol or a pharmaceutically acceptable salt thereof for the prevention, alleviation, or treatment of a muscular disease.

Hereinafter, the present invention will be described in detail

According to one aspect of the present invention, the present invention relates to a pharmaceutical composition for preventing or treating a muscular disease, which comprises inotodiol and a pharmaceutically acceptable salt thereof. Inotodiol is known as a component that is contained in a large amount in *Inonotus obliquus*, and is also known to have effects such as an anticancer effect, an immune-enhancing effect, and the like. The inotodiol of the present invention may be extracted from *Inonotus obliquus* or may be chemically synthesized. In this case, the inotodiol may be represented by the following Formula 1.

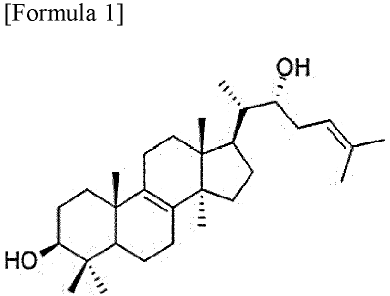

According to one aspect of the present invention, the inotodiol of the present invention has an effect of preventing or treating muscular diseases caused by muscular dysfunction, muscle loss, muscle atrophy, muscle wasting, or muscle degeneration. The term "muscular disease" refers to a condition in which muscle strength is weakened due to muscle damage or loss due to aging or diseases. In this case, the muscular disease may be caused by genetic predisposition, age-related diseases such as hypertension, impaired glucose tolerance, diabetes, obesity, dyslipidemia, atherosclerosis, cardiovascular diseases, or the like; chronic diseases such as cancer, autoimmune diseases, infectious diseases, AIDS, chronic inflammatory diseases, arthritis, malnutrition, kidney disease, chronic obstructive pulmonary disease, emphysema, rickets, chronic lower back pain, peripheral nerve damage, central nerve damage, and chemical damage; loss of motion due to causes such as fractures, trauma, and the like, or prolonged bed rest; aging, and the like.

The muscular disease may include one more muscular diseases selected from the group consisting of atony, muscular atrophy, muscular dystrophy, muscle degeneration, myotonia, amyotrophic lateral sclerosis, myasthenia, cachexia and senile sarcopenia. Specifically, the muscular disease may include muscular diseases caused by senile myoatrophy, cancer, and chronic diseases, or diseases such as muscular atrophy and the like caused by muscle disuse. More specifically, the muscular disease may include muscular atrophy, muscular dystrophy, muscle degeneration, myotonia, amyotrophic lateral sclerosis, myasthenia, cachexia, senile sarcopenia, and muscle loss, which are caused by senile myoatrophy or cancer, but the present invention is not limited thereto. In particular, in the present invention, the muscular disease may include one or more muscular diseases selected from the group consisting of muscular atrophy caused by disuse, sarcopenia caused by aging, and muscular dystrophy.

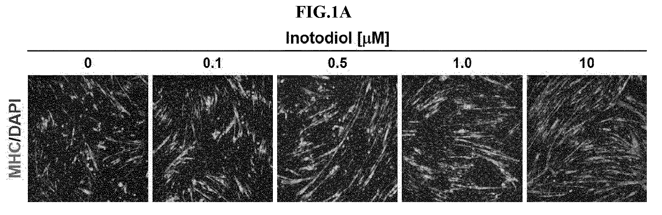

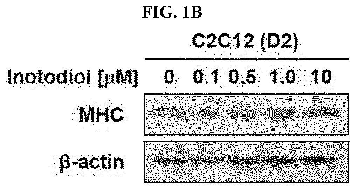

According to one aspect of the present invention, the preventive, therapeutic or ameliorative effect on the muscular disease may be achieved by promoting the differentiation of myoblasts. In one embodiment of the present invention, it was confirmed that inotodiol promotes the differentiation of myoblasts. The term "differentiation of myoblasts" refers to a process in which mononucleated myoblasts are fused to form multinucleated myotubes, and cells in the differentiation stage forming myotubes may be distinguished using markers such as Pax7-, MyoD+, myogenin, and the like. The expression of myogenic transcription factors such as MyoD increases in the cells in the differentiation stage forming myotubes, and myogenin increases in the middle stage. In the late stage of differentiation, the expression of a myosin heavy chain (MHC) increases. In one embodiment of the present invention, it was confirmed that the expression of MHC serving as a myoblast differentiation marker increases when myoblasts are treated with inotodiol (FIG. 1).

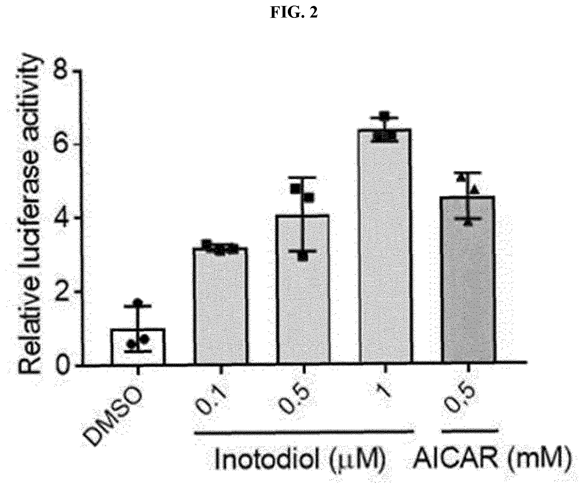

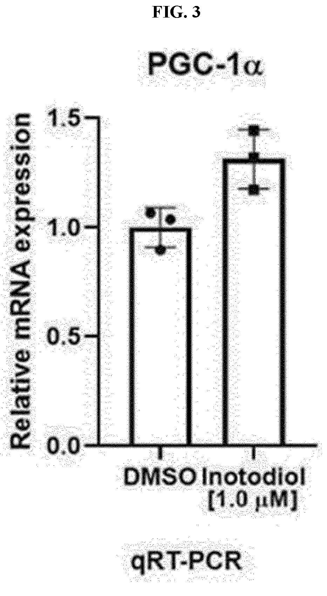

Also, in the present invention, the preventive, therapeutic or ameliorative effect on the muscular disease may be achieved by enhancing mitochondrial activity in muscle. Muscle growth and muscular endurance may be improved through mitochondrial activity in muscle, thereby improving muscle strength. In addition, the enhancement of mitochondrial activity in muscle may results in an effect of increasing the expression of PGC-1α or increasing an expression level of MHC type IIb+ muscle fibers in muscle. In one embodiment of the present invention, it was experimentally confirmed that the activity and expression of PGC-1α, which is a marker associated with mitochondrial activity, increases when myoblasts were treated with inotodiol (FIGS. 2 and 3).

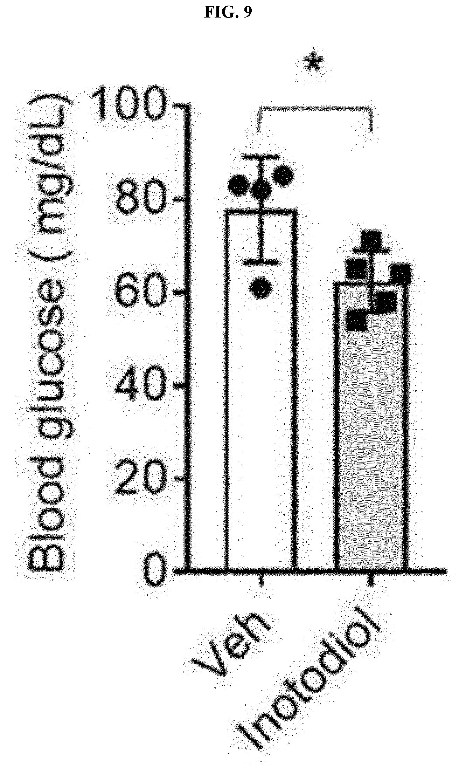

Also, inotodiol may promote muscle metabolism by promoting mitochondrial activity in muscle as described above. As a result, inotodiol may have a blood sugar-lowering effect (FIG. 9).

Therefore, due to the above effects, the inotodiol compound of the present invention may regenerate the damaged muscles of a mouse and have a preventive, therapeutic, or alleviative effect on muscle diseases.

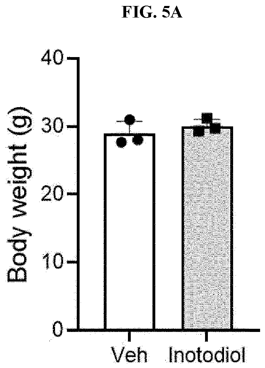

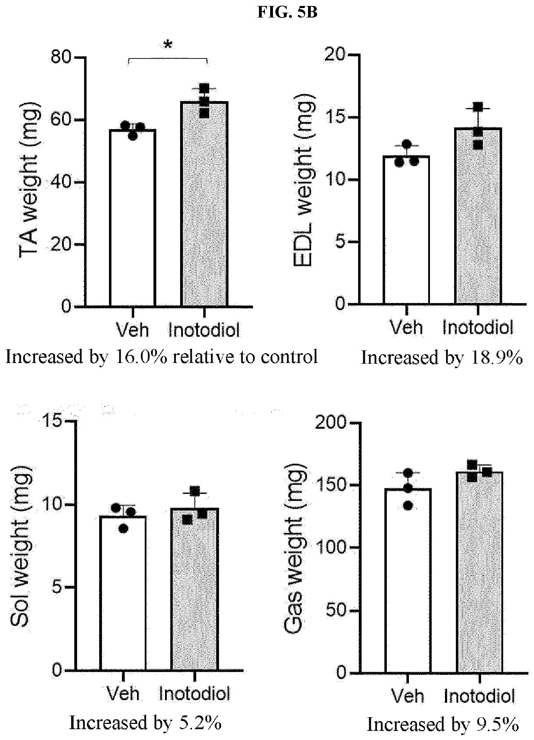

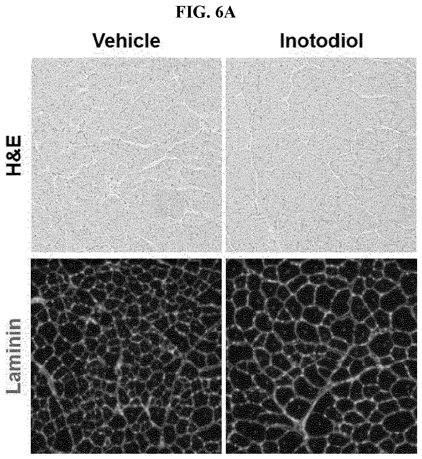

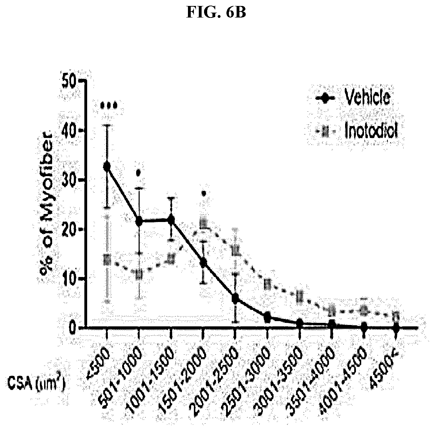

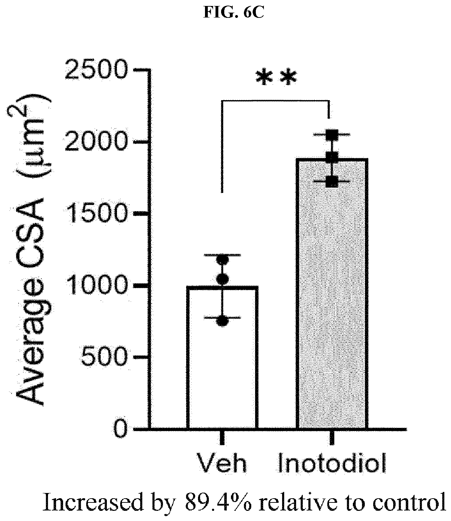

In addition, according to one embodiment of the present invention, the effects achieved by administering inotodiol to a muscle-damaged mouse model (CTX-injury) were confirmed. As a result, when compared to the control (a vehicle-administered group), although it was confirmed that no change in body weight was observed, hind-limb muscle mass relatively increased (FIG. 5). When the degree of regeneration of muscles and muscle fibers damaged by cardiotoxin (CTX) was evaluated in terms of the size of muscle fibers, and the like, it was confirmed that the muscle fiber cross-sectional area (CSA) increased 89.4% on average in the inotodiol-administered group compared to the control (FIG. 6).

The inotodiol compound of the present invention has a muscle strength-enhancing effect. The exercise performance ability may be improved by enhancing muscle strength. In this case, the term "exercise performance ability" refers to an ability to perform exercise with muscle strength. The muscle strength may be improved by increasing an amount of muscle, muscle endurance, oxidative muscle mass, promoting muscle recovery, and improving the energy balance in muscle, and may also be enhanced by reducing fatigue substances in muscle, and the like. The inotodiol compound of the present invention particularly has an effect of enhancing muscle strength by enhancing muscle self-renewal, differentiating muscle cells, increasing muscle mass, regenerating muscles, and the like.

Based on the above effects, in one embodiment of the present invention, it can be seen through a grip test that the actual exercise capacity increased in a group in which inotodiol is administered to a muscle-damaged mouse model (CTX-injury), compared to the control (a vehicle-administered group).

The composition of the present invention may be used for various purposes such as pharmaceuticals, health functional foods, functional foods, animal feed, cell culture compositions, and the like, and has an effect of promoting the differentiation of myogenic cells or enhancing mitochondrial activity in muscle.

As used in this specification, the term "prevention" refers to all actions capable of suppressing or delaying the onset of muscle diseases by administration of the pharmaceutical composition according to the present invention.

As used in this specification, the term "treatment" refers to all actions that improve or beneficially change the symptoms of muscle diseases by administration of the pharmaceutical composition according to the present invention.

The pharmaceutical composition of the present invention may be formulated and used in the form of oral formulations such as powders, granules, tablets, capsules, suspensions, emulsions, syrups, aerosols, and the like; external preparations, suppositories, and sterile injection solutions according to conventional methods, and may further include carriers or excipients necessary for the formulation. Pharmaceutically acceptable carriers, excipients and diluents that may be further included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, magnesium stearate, mineral oil, and the like. When formulated, the pharmaceutical composition is prepared using diluents or excipients such as commonly used fillers, extenders, binders, wetting agents, disintegrants, surfactants and the like.

For example, a solid preparation for oral administration includes a tablet, a pill, a powder, granules, a capsule, and the like. Such a solid preparation is prepared by mixing the extract or compound with at least one excipient, for example, starch, calcium carbonate, sucrose or lactose, gelatin, or the like. Also, lubricants such as magnesium stearate, talc, and the like are used in addition to simple excipients. A liquid preparation for oral administration may include a suspension, an oral liquid, an emulsion, syrup, and the like. In addition to commonly used simple diluents such as water, liquid paraffin, and the like, the liquid preparation may include various excipients, for example, a wetting agent, a sweetening agent, a fragrance, a preservative, and the like.

A preparation for parenteral administration includes a sterile aqueous solution, a nonaqueous solvent, a suspending agent, an emulsifying agent, a freeze-dried preparation, a suppository, and the like. Propylene glycol, polyethylene glycol, a vegetable oil (such as olive oil), an injectable ester (such as ethyl oleate), and the like may be used as a nonaqueous solvent and a suspending agent. Witepsol, Macrogol, Tween 61, cacao butter, laurin butter, glycerogelatin, and the like may be used as a base of the suppository.

The pharmaceutical composition of the present invention may be administered orally or parenterally (by intravenous injection, subcutaneous, intraperitoneal, or topical application) according to desired methods. A dose of the pharmaceutical composition may vary depending on the condition and weight of a patient, the severity of a disease, the type of drug, and the route and time of administration, and may be selected in an appropriate form by those skilled in the art.

The pharmaceutical composition of the present invention is administered in a pharmaceutically effective amount. In the present invention, the term "pharmaceutically effective amount" refers to a reasonable amount applicable to medical treatment, that is, an amount sufficient to treat a disease. In this case, the criterion for the effective amount may be determined according to a patient's disease, the severity of the disease, the activity of a drug, the sensitivity to the drug, the time of administration, the route of administration, and the excretion rate, the duration of treatment, factors including drugs used concurrently, and other factors. The pharmaceutical composition of the present invention may be administered as an individual therapeutic agent or in combination with other therapeutic agents, and may be administered sequentially or concurrently with conventional therapeutic agents. The dosage may be determined at a level that may minimize side effects in consideration of all of the above factors, which may be easily determined by those skilled in the art. Specifically, the dosage of the pharmaceutical composition may vary depending on the age, weight, and gender of a patient, the severity of a disease, and the like. In general, the pharmaceutical composition may be administered daily, every other day, or 1 to 3 times a day at a dose of 0.001 to 150 mg, more preferably 0.01 to 100 mg per 1 kg of body weight. However, this is given for exemplary purposes, and the dosage may be set differently when necessary.

Also, the composition of the present invention may be a food or a health functional food. In particular, the term "health functional food" refers to a food prepared or processed using raw materials or components having useful functionalities for the human body according to the Health Functional Food Act No. 6727, and the term "functional" means that a food is consumed for the purpose of adjusting nutrients for the structure and functions of the human body or obtaining useful effects for health purposes such as physiological functions, and the like.

The food or health functional food of the present invention may be prepared or processed into pharmaceutical dosage forms such as powders, granules, tablets, capsules, pills, suspensions, emulsions, syrups, and the like; or health functional foods such as tea bags, leachates, beverages, candies, jellies, gum, and the like for the purpose of preventing and improving muscle diseases.

The food or health functional food composition of the present invention may be used as a food additive, and may be commercialized alone or in combination with other ingredients. Also, the food or health functional food composition may include nutrients, vitamins, electrolytes, flavoring agents, coloring and enhancing agents, pectic acid and salts thereof, alginic acid and salts thereof, organic acids, protective colloidal thickeners, pH regulators, stabilizers, preservatives, glycerin, alcohol, carbonating agents used for carbonated beverages, and the like. The components may be used alone or in combination, and may be used in combination in a suitable content.

According to another aspect of the present invention, the present invention relates to a feed or feed additive composition comprising an inotodiol compound.

In the present invention, the term "feed" refers to a material that supplies organic or inorganic nutrients necessary for maintaining the life of an animal. The feed includes nutrients such as energy, proteins, lipids, vitamins, minerals, and the like required by animals such as livestock, and the like, and may include vegetable feed or proteins such as grains, roots and fruits, food processing by-products, algae, fibers, fats and oils, starches, gourds, cereal by-products, and the like; animal feed such as inorganic materials, fats and oils, minerals, and single-cell proteins, but the present invention is not limited thereto.

In the present invention, the term "feed additive" refers to a substance added to the feed to improve the productivity or health of animals, but the present invention is not limited thereto. In this case, the feed additive may further include amino acids, vitamins, enzymes, flavors, silicates, buffers, extracts, oligosaccharides, and the like for the purpose of promoting the growth of animals and preventing diseases, and the like. The feed or feed additive composition of the present invention may have an effect of promoting the differentiation of myogenic cells in animals, regenerating muscle, or enhancing muscle strength, and may further have an effect of preventing, treating, or improving muscle-related diseases in animals.

In another aspect, the present invention relates to a composition for enhancing muscle self-renewal, promoting the differentiation of muscles, or increasing muscle regeneration or muscle mass, and to a composition for enhancing muscle strength and alleviating muscle function, which comprises an inotodiol compound.

The inotodiol of the present invention has an effect of enhancing muscle self-renewal. Here, the term "enhancing muscle self-renewal" refers to the ability of muscle stem cells to maintain their own number without completely differentiating into myogenic cells. That is, muscle stem cells may maintain a certain number thorough the promotion of muscle self-renewal, indicating that muscle stem cells have an ability to continuously regenerate muscles even in the event of extrinsic damage caused by external shock or muscle damage caused by endogenous factors including chronic inflammation, and eventually maintain muscle mass.

Also, the composition of the present invention has an effect of "promoting myoblast differentiation," and the term "myoblast differentiation" refers to a process in which mononucleated myoblasts are fused to form multinucleated myotubes. Here, myoblast differentiation refers to an action of inducing this process. Cells in the differentiation stage forming myotubes are identified through the expression of markers such as Pax7-, MyoD+, MyoG+, and the like. Muscle cells increase by promoting the differentiation of myoblasts, and may ultimately bring about an effect of increasing muscle mass in terms of individuals.

In addition, the composition of the present invention has a "muscle regeneration" effect, and the term "muscle regeneration" refers to an ability of damaged muscles to recover to a normal state. According to one embodiment of the present invention, it was confirmed through experiments that muscle tissue acutely damaged by injecting cardiotoxin as a neurotoxin recovers more quickly by the administration of inotodiol. The muscle regeneration may be effected by promoting the differentiation of muscle cells to increase the absolute amount of muscle cells or increase the diameter of individual myotubes.

Further, the present invention relates to a composition for enhancing muscle strength, which comprises inotodiol. The term "muscle strength enhancement" refers to effects of enhancing physical performance, enhancing maximum endurance, increasing muscle mass, enhancing muscle recovery, reducing muscle fatigue, improving the energy balance in muscle, or a combination thereof. As described above, the composition of the present invention may increase total muscle mass by increasing muscle mass through the ability to differentiate myoblasts into muscle cells, and promote muscle regeneration to enhance maximum endurance, thereby improving physical performance and reducing muscle fatigue. Also, because muscle cells may be quickly replaced, damaged muscles may quickly heal.

The inotodiol compound of the present invention has an effect of improving muscle function. Here, the term "muscle function improvement" refers to an ability to exert a force by the contraction of muscles, and includes muscle strength, which is an ability of muscles to exert a maximum contraction force to overcome resistance, muscle endurance, which is an ability of muscles to show how long or how many times a muscle can repeat muscular contraction and relaxation with a given weight, and instantaneous power, which is an ability of muscles to exert a strong force in a short time. Such muscle function is proportional to muscle mass, and the term "muscle function improvement" refers to an action of making muscle function better. Also, because the inotodiol of the present invention enhances mitochondrial activity in muscle, the energy balance in muscle is improved accordingly. As a result, the inotodiol of the present invention may have an effect of improving muscle function.

The inotodiol compound of the present invention has an effect of alleviating muscle fibrosis, and the term "muscle fibrosis" refers to a symptom of a decrease in muscle elasticity, which appears as tendons constituting muscles harden and clump for a long time. As shown in one experimental example of the present invention, it was confirmed that the muscle fibrosis is improved by the administration of the inotodiol compound.

The composition for enhancing the self-renewal capacity of muscle stem cells, promoting the differentiation capacity of myoblasts, regenerating muscles, and increasing muscle mass, and the composition for enhancing muscle strength or alleviating muscle function according to the present invention may be prepared in the form of a food composition or a food additive, and particularly prepared in the form of a health functional food composition. Here, the food composition is as described above. Therefore, the composition for enhancing muscle strength according to the present invention may be used in the form of an adjuvant not only for the prevention of muscle loss due to aging or disease, but also for muscle generation and muscle strength enhancement in ordinary persons.

### [Mode for Invention]

Hereinafter, the present specification will be described in detail with reference to embodiments thereof in order to specifically describe the present specification. However, it should be understood that the embodiments according to the present specification may be modified in various forms, and the scope of the present specification is limited to the embodiments described below in detail. The embodiments of the present specification are provided to more completely describe the present specification to a person with ordinary skill in the art.

### Example 1: Culture and induced differentiation of myoblast cell line C2C1

C2Cl2 is a myoblast cell line obtained from the live mice of the C3H species, and has been widely used in myocyte differentiation studies. The C2C12 cells were cultured in a general cell culture medium and a differentiation medium, respectively. DMEM supplemented with 15% fetal bovine serum was used as the normal cell culture medium (i.e., a growth medium (GM)), and DMEM supplemented with 2% horse serum was used as the differentiation medium (DM).

Cells were seeded into the cell culture medium (GM), and cultured for 24 hours. Thereafter, the differentiation medium (DM) was treated with DMSO and inotodiol (0.1, 0.5, 1.0, and 10 µM) at different concentrations, and differentiation was induced for 2 to 3 days.

### Example 2: Mouse model in which muscle is damaged by CTX

Ten 5-month-old C57BL/6 male mice were used as experimental animals. Five animals each of the experimental animals having a similar body weight were assigned and classified into a control to which inotodiol was not administered and an experimental group to which inotodiol was administered. Inotodiol was dissolved in 80% saline, 10% PEG400, and 10% EtOH so that the inotodiol was prepared at a concentration of 300 µg/kg, and orally administered to the mice in the experimental group for 7 days. Thereafter, 10 µM cardiotoxin (CTX) was directly injected into the muscle at a rate of 2 µL per gram of body weight to induce muscle damage, and inotodiol (300 µg/kg) was then orally administered for 21 days.

### Example 3: Muscular dystrophy (DEX) model

Twenty 16-week-old C57BL/6 male mice were used as experimental animals. Experimental animals were grouped into 10 animals having a similar body weight, and dexamethasone was administered at a dose of 20 mg/kg to one group to induce muscular dystrophy.

### Experimental Example 1: Confirmation of myoblast differentiation-enhancing effect

The C2C12 cells whose differentiation was induced in Example 1 were washed with 1X PBS, and then fixed with 3.7% paraformaldehyde at room temperature. Thereafter, a permeabilization buffer was added thereto, and reacted at room temperature. Unspecific antibody binding was inhibited by reacting with PBST (a blocking buffer) containing 3% BSA and PBS containing 0.1% Tween 20. A primary antibody against a myosin heavy chain (MHC) was diluted 1:500 in a blocking buffer, added, and reacted at room temperature. A secondary antibody diluted 1:5000 in a blocking buffer was added, and reacted at room temperature. Then, after the cells were treated with a mounting solution and fixed, photographs were taken with a fluorescence microscope to analyze the results.

As a result, it was confirmed that the formation of multinucleated myotubes increased in a concentration-dependent manner in the case of C2C12 cells treated with inotodiol at different concentrations, as seen from an immunostaining image on day 2 of differentiation (FIG. 1 (top)).

The C2C12 cells whose differentiation was induced in Example 1 were collected, lysed by adding a lysis buffer (20 mM Tris-HCl, pH 8.0, 150 mM NaCl, 1% Triton X, a proteinase inhibitor), and then the cell lysis sample was quantified. Thereafter, the same amount of protein was subjected to SDS-PAGE electrophoresis, and transferred to a PVDF membrane. The membrane was blocked with 5% skim milk, and washed with TTBS (0.03% Tween 20, 2.42 g of Tris, 9 g of NaCl, pH 7.41/L). As the differentiation marker, a myosin heavy chain (MHC) primary antibody was diluted 1:500 in TTBS containing 5% BSA, and added. Then, the resulting mixture was reacted overnight at 4°C. Thereafter, a secondary antibody was diluted 1:5000 in TTBS containing 5% skim milk, added, and reacted at room temperature. Then, after an enhanced chemiluminescent solution (ECL, Pierce) was added thereto, the membrane was exposed to an X-ray film to determine the amount of protein.

As a result, as shown in FIG. 1 (bottom), it was confirmed that the expression level of MHC serving as a differentiation marker was increased in a concentration-dependent manner in the C2C12 cells treated with inotodiol at different concentrations, compared to the control (a DMSO-treated group). Taken together, it was confirmed that inotodiol promoted the differentiation of myoblasts.

### Experimental Example 2: PGC-1α reporter assay

Immediately after cells were seeded at a density of 1 × 10⁴ cells/well in a 96-well culture plate, the cells were transfected by treating the cells with PGC-1α (DNA) and a plasmid at a ratio of [150 ng/10 µL] + [0.3 µL/10 µL] per well. After 24 hours, the differentiation medium was treated with inotodiol at different concentrations (0, 0.1, 0.5, and 1.0 µM), and cell differentiation was induced for 24 hours. After 24 hours of drug treatment, the cells were lysed in 30 µL of a passive lysis buffer (Promega) per well, and the dissolved cell solution was transferred to a plate for luminometer reading. Then, an equal amount of luciferin, which is a substrate of luciferase, was subjected to an enzymatic reaction to determine the degree of luminescence. The measured values were standardized for each well and plate, and expressed as a relative ratio using the measured values in the wells treated with DMSO, which is a solvent of the compound, as the control. The expression level of luciferase in the PGC-1α reporter cell line depends on the stimulation received by a PGC-1α promoter. That is, if the compound is a PGC-1α expression inducer, it stimulates the PGC-1α promoter to increase the expression of luciferase, and if the compound is a PGC-1α expression inhibitor, luciferase expression decreases. As for the expression level, the degree of activation or inhibition of PGC-1α transcription by inotodiol was measured by measuring the luminescence produced by the addition of a luciferase substrate.

As a result, as shown in FIG. 2, it can be seen that the luciferase activity for PGC-1α increased in an inotodiol concentration-dependent manner in the group treated with inotodiol. Also, it was confirmed that the luciferase activity for PGC-1α was higher than that of 0.5 mM AICAR used as a positive control.

### Experimental Example 3: Analysis of PGC-1α mRNA expression by qRT-PCR

The C2C12 cells whose differentiation was induced in Example 1 were lysed using an Easy-spin Total RNA Extraction kit, and chloroform was added thereto. Then, the C2C12 cells were centrifuged. After the supernatant was separated, the same volume of isopropanol was added and bound to the cells. After centrifugation, the cells were washed with 70% EtOH mixed with DEPC water, and the concentration of RNA was quantified by adding 20 to 50 µL of a DEPC solution. RNA was reverse-transcribed using a PrimeScript cDNA synthesis kit to synthesize cDNA. Then, the gene expression was analyzed by qRT-PCR in a real-time PCR system using SYBR Premix EX Taq. Fold changes in gene expression were normalized against the expression of ribosomal gene 18s rRNA.

As a result, as shown in FIG. 3, it was confirmed that the mRNA expression for PGC-1α in the inotodiol-treated group significantly increased compared to the control. Taken together, it was confirmed that inotodiol increased both the enzyme activity and expression of PGC-1α during the differentiation of myoblasts.

### Experimental Example 4: Mitochondrial activity-enhancing effect

To induce the differentiation of C2C12 myoblasts in the same manner as in Example 1 and check mitochondrial activity, the expression characteristics of a T-OxPHOS antibody mixed with ATP5A, MTCO1, and NDUF88 were confirmed through immunoblotting. For each of the proteins included in the T-OxPHOS antibody, ATP5A is a constituent protein of ATP synthase that contributes to the ATP synthesis in mitochondria, MTCO1 is associated with the oxidase activity of cytochrome-c, which is a component of the electron transport system in the inner membrane of mitochondria, and NDUF 88 encodes a subunit of NADH:ubiquinone oxidoreductase (Complex 1), which is the first enzyme complex in the mitochondrial electron transport system.

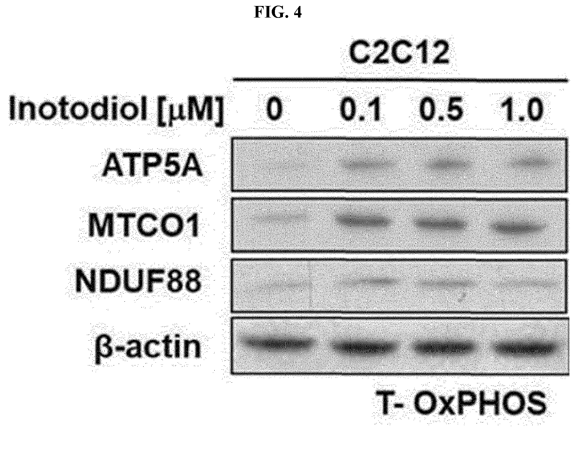

As a result, as shown in FIG. 4, it was confirmed that the expression level of all the proteins increased in proportion to the concentration of inotodiol, indicating that mitochondrial activity in muscle is enhanced by inotodiol.

### Experimental Example 5: Effect of improving muscle function in muscle-damaged mouse model (by CTX administration)

### 5-1. Confirmation of effect of increasing muscle mass

After 21 days of administration of inotodiol to the mouse model in which muscle damage was induced by CTX as in Example 2, the body weights and hindlimb muscle weights of the mice were measured. As a result, as shown in FIG. 5A, no change in body weight was observed in the experimental animals when inotodiol was administered in the mouse model in which muscle damage was induced by CTX. However, each of the weights of the hindlimb muscle tissues, such as the tibialis anterior muscle (TA), extensor digitorum longus muscle (EDL), soleus muscle (Sol), and gastrocnemius muscle (Gas), of the experimental animals was measured. As a result, it was confirmed that the muscle mass increased by 16.0% in TA, 18.9% in EDL, 5.2% in Sol, and 9.5% in Gas, respectively, in the inotodiol-administered experimental group compared to the control (FIG. 5B). In particular, the TA muscle mass increased by approximately 16.0%, indicating that the regenerative ability of muscles damaged by CTX was further enhanced by the administration of inotodiol.

### 5-2. Confirmation of effects of regenerating muscle and increasing muscle fiber size

Hematoxylin & eosin staining (H&E staining) was performed using the TA muscle isolated from the tissue of each of the experimental animals. Frozen sections fixed with 4% paraformaldehyde were stained with hematoxylin and stained with eosin for contrast staining, mounted, and observed under an optical microscope. For the analysis of muscle fiber size, immunohistochemical staining was also performed on TA muscle tissue using a laminin antibody, and observed under a fluorescence microscope.

As a result of H&E staining, as shown in FIG. 6, it was confirmed that a significant muscle regeneration effect was observed when inotodiol was administered to the mice whose muscle damage was induced by CTX compared to the control (FIGS. 6A and 6B). Also, the cross-sectional area (CSA) of the TA muscle was analyzed through laminin staining. As a result, it was confirmed that the percentage of muscle fibers having a large cross-sectional area in the TA muscle increased in the inotodiol-administered group compared to the control. In particular, the large cross-sectional area percentage of the entire TA muscle increased by 89.4% compared to the control (FIG. 6C).

### 5-3. Changes in types of skeletal muscle fibers by administration of inotodiol

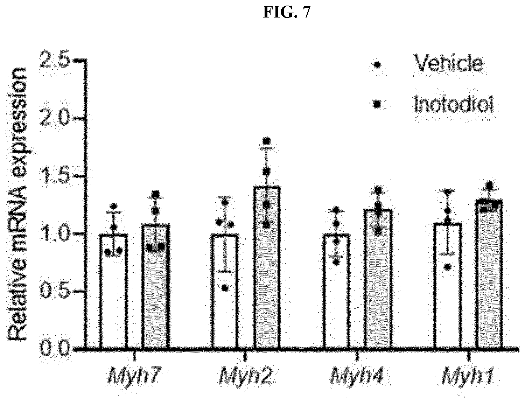

In the CTX-injected mouse model, when inotodiol was administered 21 days after muscle damage, changes in types of skeletal muscle fibers were observed. The relative mRNA expression levels were compared for each muscle type of Myh7 (MHCI), Myh2 (MHCIIa), Myh4 (MHCIIb), and Myh1 (MHCIIx). As a result, there was no significant difference as shown in FIG. 7, but the expression levels generally tended to increase. In particular, it can be confirmed that the expression of a glycolytic myofiber Myh2 (MHCIIa) tended to greatly increase.

### 5-4. Confirmation of effect of increasing muscle strength

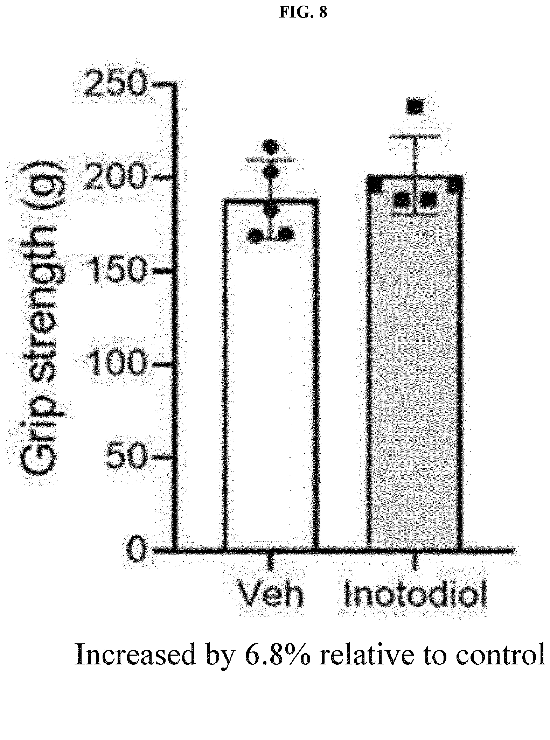

On day 21 after inducing muscle damage by CTX, a grip strength test was performed in order to check the muscle strength of the inotodiol-administered group and the control. The grip strength test was performed using a commercially available mouse grip strength tester manufactured by BIOSEB. The mouse was placed on a wire mesh attached to an instrument panel capable of monitoring the strength of the force, and the force for the mouse to grip the wire mesh was measured while grabbing the mouse tail and pulling it downward. The average value obtained by successively repeating the above process 5 times was used. As a result, it was confirmed that the grip strength increased by approximately 6.8% in the inotodiol-administered group compared to the control (FIG. 8).

### 5-6. Blood glucose-lowering effect

When inotodiol was administered to the experimental animals whose muscle damage was induced by CTX, the amount of blood glucose change was measured. The improvement of mitochondrial functions in muscle is closely associated with the activation of energy metabolism in muscle, and a blood glucose-lowering effect may be induced as a side effect. When the blood glucose levels of the experimental animals were analyzed on day 21 after the induction of muscle damage by CTX, the blood glucose-lowering effect was confirmed in the inotodiol-administered group compared to the control (FIG. 9).

The fact that blood glucose was lowered in the inotodiol-administered group may be inferred to result from increased consumption of glucose in muscle due to mitochondrial activity in muscle. Because the mitochondrial activity in muscle was enhanced as shown in Experimental Examples 2 and 3 when inotodiol is administered, the blood glucose-lowering effect is due to the increased consumption of blood glucose in mitochondria in muscle. For another reason, because the differentiation and regeneration of muscle are promoted by the administration of inotodiol, absolute muscle mass increases, which may be seen as a result of increased glucose consumption by the energy metabolism in muscle.

### 5-7. Effect of promoting stem cell proliferation by administration of inotodiol

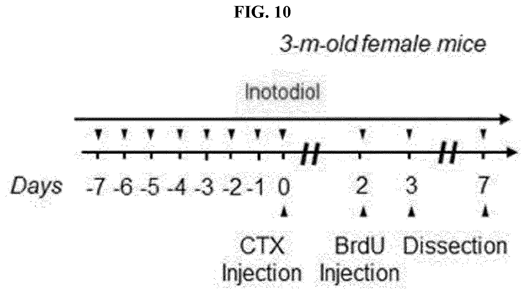

To check the muscle regeneration effect when inotodiol was administered to a mouse model in which muscle damage was induced by injecting CTX, an experiment was conducted. To induce muscle damage, CTX was injected into the mice to which inotodiol was administered once a day for 7 days. Then, it was determined through BrdU analysis whether or not the proliferation of muscle stem cells was enhanced (FIG. 10).

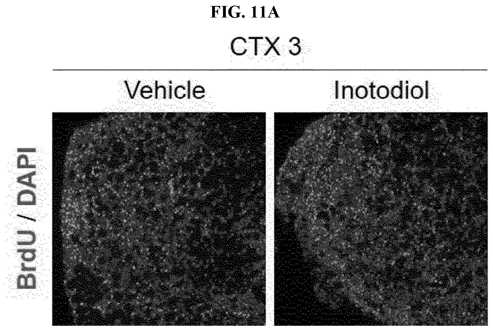

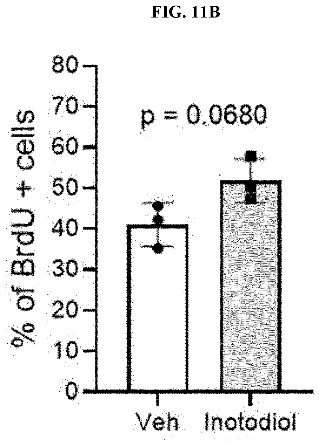

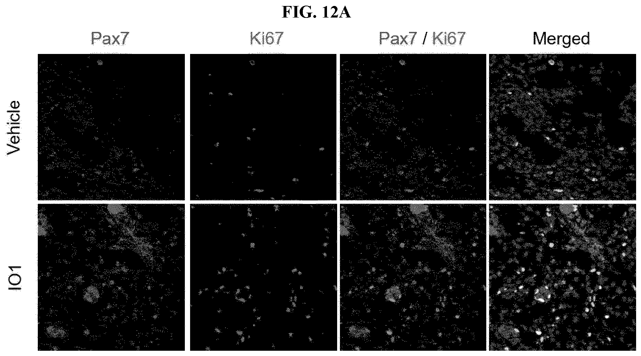

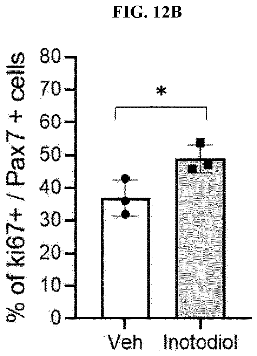

As a result, as shown in FIGS. 11A and 11B, it was confirmed that the expression level of BrdU increased in the muscle tissues of the inotodiol-administered mice. As shown in FIGS. 12A and 12B, it was confirmed that the expression levels of Pax7 and Ki67 and the expression ratio of Ki67 to Pax7 increased.

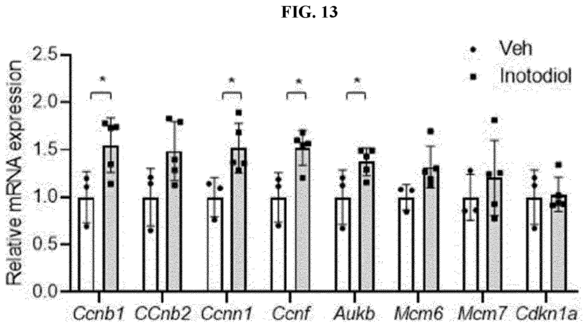

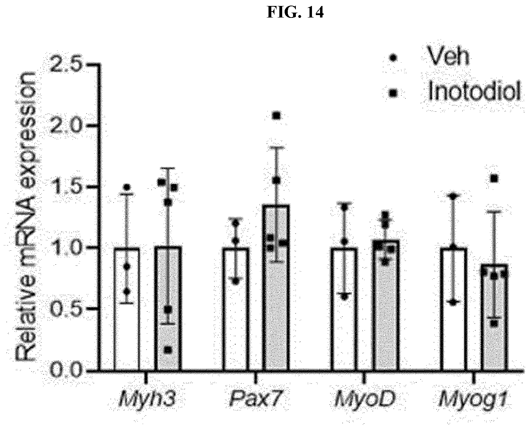

Also, as shown in FIG. 13, it can be confirmed that the relative mRNA expression levels of genes involved in the cell cycle increased, and the effect of enhancing the expression of genes involved in muscle growth was confirmed, as shown in FIG. 14.

Based on the above results, it can be seen that the proliferation of muscle stem cells was promoted by inotodiol.

### 5-8. Inhibitory effect on muscle fibrosis by administration of inotodiol

To check the effect on muscle fibrosis when inotodiol was administered to a mouse model in which muscle damage was induced by injecting CTX, an experiment was conducted. CTX was injected for 7 days to induce muscle damage, and inotodiol was then administered to confirm whether muscle fibrosis was ameliorated.

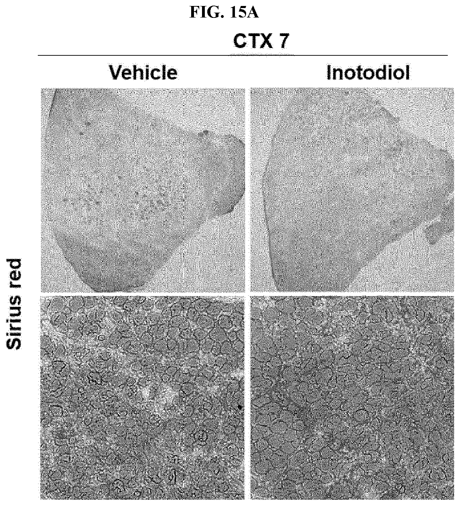

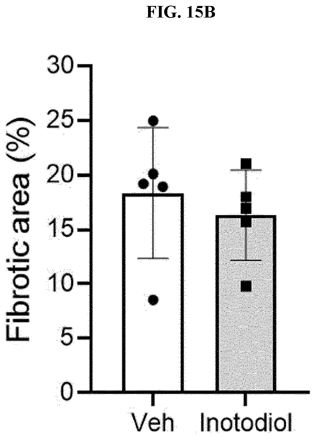

As a result, as shown in FIGS. 15A and 15B, it was confirmed that fibrosis was ameliorated in the muscle tissue to which inotodiol was administered, and the area of the muscle tissue in which fibrosis progressed was reduced.

### Experimental Example 6: Effect of improving exercise capacity in high-fat diet model

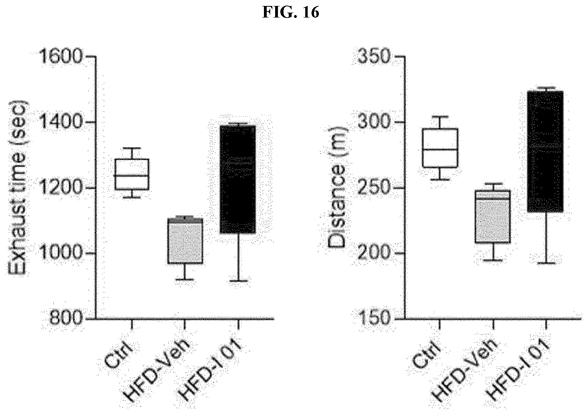

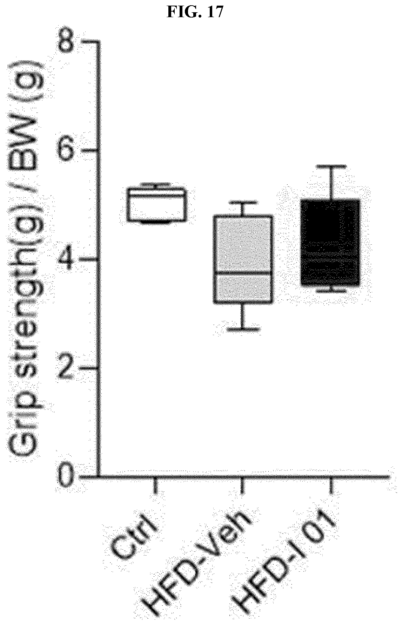

When inotodiol was administered to high-fat diet mice, the effects on muscle strength and exercise performance ability were determined. After inotodiol was administered to a high-fat diet-fed mouse model, the exercise duration and movement distance were measured through a treadmill experiment. As a result, it was confirmed that the inotodiol-administered group, on average, recovered exercise capacity to the level of the normal control (FIG. 16). As a result of the grip test, it was also confirmed that the high-fat diet mouse model had lower muscle strength than the mean muscle strength. However, it was confirmed that muscle strength was improved by the administration of inotodiol (FIG. 17).

### Experimental Example 7: Effect of alleviating myotube atrophy in myoatrophy-induced (DEX) model

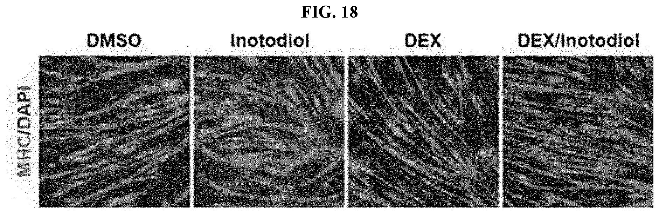

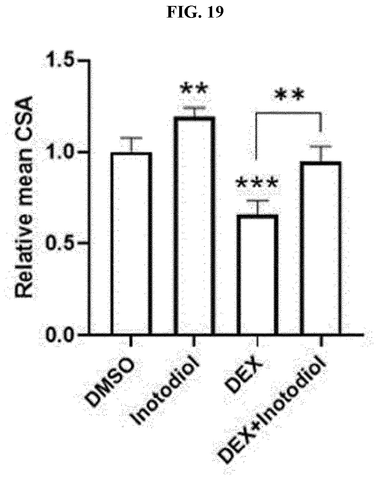

For the myoatrophy mouse model of Example 3, the effects of alleviating and recovering myotube atrophy were determined when inotodiol was administered. As a result, it was confirmed that the size of muscles and muscle fibers increased. Based on the results obtained through the cross-sectional area of myotubes, it can be seen that the cross-sectional area of the myotubes was restored to the same level as that of the normal group when inotodiol was administered to the model in which myotube atrophy induced by DEX (FIGS. 18 and 19).

### Experimental Example 10: Effects of increasing muscle mass and strength in long-term inotodiol-administered model

### 10-1. Effect of increasing muscle mass

For normal mice in which the muscle damage or muscle atrophy was not induced, an experiment was conducted to determine whether there was an effect of increasing muscle mass and muscle strength when inotodiol was administered. For the mice to which inotodiol was administered for 2 months, an experiment was conducted to measure a change in muscle mass and muscle strength of hindlimb muscles after the administration of inotodiol (a grip test).

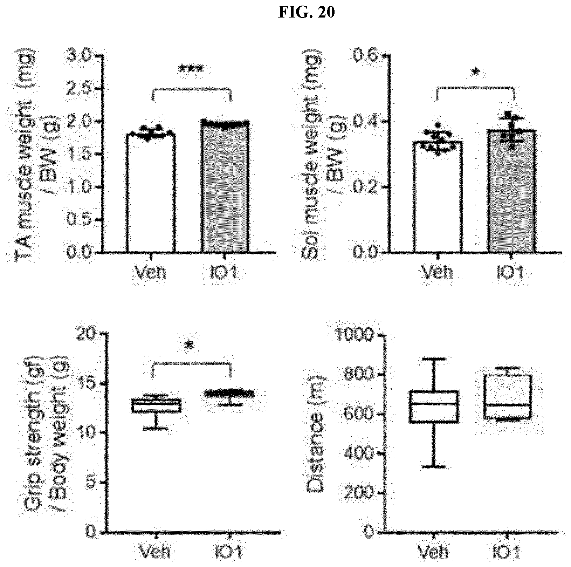

As a result, as shown in FIG. 20, it can be seen that the hindlimb muscle mass increased and muscle strength also increased compared to the control.

### 10-2. Effect of enhancing expression of PGC-la and mitochondria-related genes in muscle

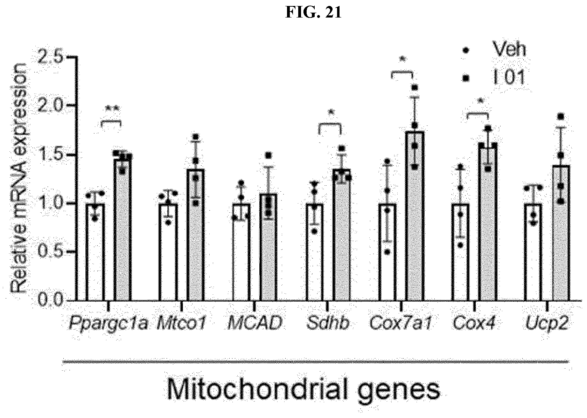

As a result of examining the expression levels of PGC-1a, which is a muscle metabolism marker, and mitochondrial-related genes in muscle upon long-term administration of inotodiol in normal mice, as shown in FIG. 21, it can be seen that the expression levels increased compared to the control.

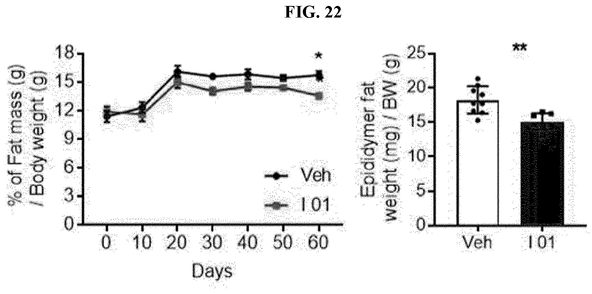

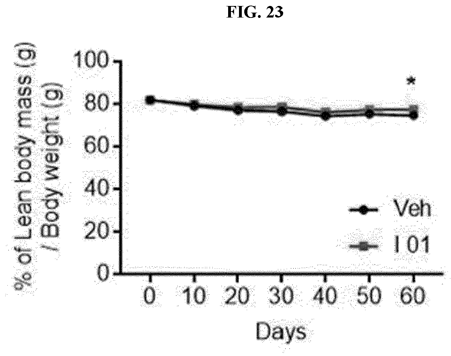

Also, a change in weight of fat relative to the weight gain in the normal mice was determined. As a result, as shown in FIG. 22, it can be seen that the weight of fat increased relatively less in the inotodiol-administered group. Also, as shown in FIG. 23, it can be seen that the weight-to-muscle ratio was higher than that of the non-administered group.

That is, the results show that muscle mass and the expression of mitochondria in muscle increased in the normal muscle cells in addition to the muscle cells having a muscular disease and the inotodiol had an effect of improving muscle strength.

In the foregoing, the present invention has been described with reference to preferred embodiments thereof. Those skilled in the art to which the present invention pertains will be able to understand that the present invention may be implemented in a modified form without departing from the essential features of the present invention. Therefore, the disclosed embodiments should be considered not from a restrictive viewpoint but from an explanatory viewpoint. The scope of the present invention is defined in the claims rather than in the above-described description, and it should be interpreted that all the differences within a range equivalent thereto are included in the present invention.

## Claims

1. A pharmaceutical composition for preventing or treating a muscular disease, comprising an inotodiol compound and a pharmaceutically acceptable salt thereof.

2. A health functional food composition for preventing or alleviating a muscular disease, comprising an inotodiol compound or a sitologically acceptable salt thereof.

3. An animal feed composition for preventing or alleviating a muscular disease, comprising an inotodiol compound or a salt thereof.

4. The composition of any of claims 1 to 3, wherein the muscular disease is selected from the group consisting of atony, muscular atrophy, muscular dystrophy, muscle degeneration, myotonia, amyotrophic lateral sclerosis, myasthenia, cachexia, muscle loss, and sarcopenia.

5. The composition of any of claims 1 to 3, wherein the muscular disease is caused by aging, muscle decline, muscle wasting, muscle degeneration, disuse, or muscle damage.

6. The composition of any of claims 1 to 3, wherein the muscular disease is sarcopenia caused by cancer or aging.

7. The composition of any of claims 1 to 3, wherein the muscular disease is muscular atrophy caused by disuse of skeletal muscle.

8. The composition of any of claims 1 to 3, wherein the composition promotes mitochondrial activity in muscle.

9. The composition of any of claims 1 to 3, wherein the composition lowers blood glucose by promoting mitochondrial metabolism in muscle.

10. A composition for enhancing the self-renewal of muscle stem cells, promoting muscle differentiation, regenerating muscles, or increasing muscle mass, comprising an inotodiol compound.

11. A composition for improving muscle function, comprising an inotodiol compound.

12. A composition for enhancing muscle strength, comprising an inotodiol compound.

13. A composition for improving exercise performance ability, comprising an inotodiol compound.

14. A composition for alleviating muscle fibrosis, comprising an inotodiol compound.

15. The composition of any of claims 10 to 14, wherein the composition is one or more selected from a food, a functional food, a health functional food, a pharmaceutical, an animal feed, or a feed additive.

16. The composition of any of claims 10 to 14, wherein the composition is administered to an injured or aged subject.

17. The composition of any of claims 10 to 14, wherein the composition is administered to a normal subject.

18. A method of preventing, alleviating, or treating a muscular disease, comprising:
administering inotodiol or a pharmaceutically acceptable salt thereof to a subject.

19. A use of the inotodiol or a pharmaceutically acceptable salt thereof for the prevention, alleviation, or treatment of a muscular disease.
